(19) Europäisches Patentamt
European Patent Office
Office européen des brevets

(11) Publication number: **0 210 227**
**B1**

# (12) EUROPEAN PATENT SPECIFICATION

(45) Date of publication of patent specification: **02.05.90**

(51) Int. Cl.$^5$: **A 61 K 7/06,** A 61 K 31/505

(21) Application number: **86900936.5**

(22) Date of filing: **23.01.86**

(86) International application number:
**PCT/US86/00073**

(87) International publication number:
**WO 86/04231 31.07.86 Gazette 86/17**

## (54) PYRIMIDINE SULFATES FOR HAIR GROWTH.

(30) Priority: **25.01.85 US 695109**

(43) Date of publication of application:
**04.02.87 Bulletin 87/06**

(45) Publication of the grant of the patent:
**02.05.90 Bulletin 90/18**

(84) Designated Contracting States:
**AT BE CH DE FR GB IT LI LU NL SE**

(56) References cited:
**US-A-4 139 619**
**US-A-4 287 338**

(73) Proprietor: **THE UPJOHN COMPANY**
**301 Henrietta Street**
**Kalamazoo, Michigan 49001 (US)**

(72) Inventor: **MC CALL, John, M.**
**3822 Edinburgh**
**Kalamazoo, MI 49007 (US)**

(74) Representative: **Perry, Robert Edward et al**
**GILL JENNINGS & EVERY 53-64 Chancery Lane**
**London WC2A 1HN (GB)**

**EP 0 210 227 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to the use of pyrimidine sulfates in hair growth.

There are many different types of hair loss, the most common being "alopecia" wherein human males begin losing scalp hair at the temples and on the crown of the head as they get older. While this type of hair loss is largely confined to males, hence its common name "male pattern baldness," it is not unknown in women. Hair loss can be seen as gradual hair conversion.

Terminal hairs are coarse, pigmented, long hairs in which the bulb of the hair follicle is seated deep in the dermis. Vellus hairs are fine, thin, non-pigmented short hairs in which the hair bulb is located superficially in the dermis. As alopecia progresses, a transition takes place in the area of approaching baldness wherein the hairs themselves are changing from the terminal to the vellus type.

All hair, both human and animal, passes through a life cycle that includes three phases, namely, (1) the anagen phase (2) the catagen phase and (3) the telogen phase. The anagen phase is the period of active hair growth and, insofar as scalp hair is concerned, this generally lasts from 3—5 years. The catagen phase is a short transitional phase between the anagen and telogen phases which, in the case of scalp hair, lasts only 1—2 weeks. The final phase is the telogen phase, which, for all practical pusposes, can be denominated by a "resting phase" where all growth ceases and the hair eventually is shed preparatory to the follicle commencing to grow a new one. Scalp hair in the telogen phase is also relatively short-lived, some 3—4 months elapsing before the hair is shed and a new one begins to grow.

Under normal hair growth conditions on the scalp, approximately 88% of the hairs are in the anagen phase, only 1% in catagen and the remainder in telogen. With the onset of male pattern baldness, a successively greater proportion of the hairs are in the telogen phase with correspondingly fewer in the active growth anagen phase.

The remaining result associated with alopecia is the severe dimunition of hair follicles. A bald human subject will average only about 306 follicles/cm$^2$, whereas a non-bald human in the same age group (30—90 years) will still have an average of 460 follicles/cm$^2$. This amounts to a one-third reduction in hair follicles which, when added to the increased proportion of vellus hair follicles and the increased number of hair follicles in telogen, is both significant and noticeable. Thus, "baldness" is produced by a combination of these factors: (1).transition of hairs from terminal to vellus, (2) increased number of telogen hairs — some of which have been shed, and (3) loss of hair follicles.

Minoxidil (6-amino-1,2-dihydro-1-hydroxy-2-imino-4-piperidinopyrimidine) is currently being developed as a hair growth agent for use in the treatment of male pattern baldness.

US—A—4287338 discloses pyrimidine sulfates or inner salts, of formula I (below), including minoxidil sulfate, and pharmaceutical compositions containing them. US—A—4139619 discloses the use of certain N-oxides, corresponding to the sulfates of formula I, to grow hair.

According to the present invention, compounds of formula I are used for the manufacture of a medicament for use in arresting or reversing male pattern baldness in a human, or for use in increasing the rate of terminal hair growth in a mammal. Formula I is

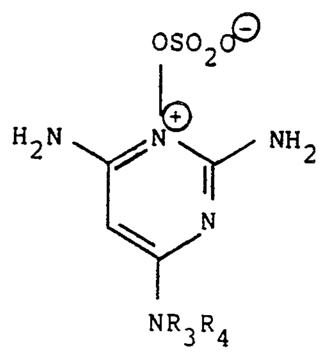

I

wherein either $R_3$ and $R_4$ are independently selected from hydrogen, lower alkyl, lower alkenyl, lower aralkyl and lower cycloalkyl, or $NR_3R_4$ is a heterocyclic moiety selected from aziridinyl, azetidinyl, pyrrolidinyl, piperidino, hexahydroazepinyl, heptamethylenimino, octamethylenimino, morpholino and 4-(lower alkyl)piperazinyl, the heterocyclic moiety optionally having, on its carbon atoms, up to 3 substituents selected from lower alkyl, hydroxy and alkoxy; and includes pharmacologically-acceptable acid addition salts thereof.

The preparation of compounds of formula I is described in US—A—4287338, as are oral parenteral formulations of the compounds. For use in the invention, compounds of formula I may be amdinistered orally or parenterally, but topical administration, preferably twice daily, is preferred. Suitable topical compositions, and their use, are described in US—A—4139619.

The amount of the compound of formula I in a composition to be used in accordance with the present invention is preferably from 0.05 to 20, more preferably from 0.1 to 2% by weight.

For use in the present invention, the compounds of formula I may also be formulated into gels, or into

conventional liposomal preparations. The compounds may also be dissolved in conventional solvents such as dimethyl sulfoxide, acetonitrile, dimethylformamide, dimethylacetamide or propylene glycol/ethanol/water. The last solvent is preferred.

2,6-Diamino-4-(1-piperidinyl)-1-(sulfoxy)-pyrimidinium hydroxide, inner salt, also known as minoxidil sulfate, is the most preferred compound for use in this invention. It is preferred to apply the compound to the scalp for the treatment or prevention of male pattern baldness.

The present invention is illustrated by the Examples, below. Examples 1—3 illustrate the effect of minoxidil sulfate (a compound of the present invention) and minoxidil (the corresponding N-oxide) in genetically identical hair-deficient mice called "nude" (nu/nu) (see Flanagen, Genet. Res. 8:295—309 (1966)). In this mutant, the mouse exhibits only a narrow band of hair which moves from the head to the tail of the mouse throughout a 20 day cycle. All mice in the species *mus* grow hair in a definite, 20 day cycle, going from head to tail (see Barom, Acta Path. Micro. Scan. 34: 521—541 (1954)). "Nude" mice have the same pattern (see Eton, Transplantation 22: 217—222 (1976)). Hair fails to appear in the nude mouce since it never emerges due to the fact that the hair shaft is incompletely formed, poorly keratinized, or both. The nacent hair shafts fragment within the follicle and thus never emerge or break near the skin surface. The hair produced in the nude mice treated with the compounds described below is more normal and mechanically competent to emerge and persit. Eventually, however, even the hair produced in the drug treated animals falls out due to the genetic defect of these animals. Nonetheless, significant differences in hair growth can be observed in the drug treated animals as compared to the control animals during the twenty day cycle.

In the Examples, five photographs were taken of the animals every six days over a four week period. The results are set forth in Table I. Animals exhibiting significant amounts of hair in at least one or two days are tabulated therein.

## Example 1

Nine week old Balb/c, nu/nu, male mice were given oral drug daily except weekends suspended in 0.25 ml water. Actual weights were between 20—30 g and an average of 25 g was assumed for dosage purposes.

## Example 2

Eleven week old mice from the same group fo animals as used in Example 1 were treated by oral administration of 0.25 ml of either minoxidil or minoxidil sulfate in water daily without resting on weekends. Weights were assumed to be an average of 25 g per animal for dosing purposes.

## Example 3

Six week old Balb/c nu/nu male mice were given daily oral drug (without resting on weekends) in 0.25 ml water. Topical minoxidil sulfate was applied in 0.25 ml of a propylene glycol, ethanol, and water vehicle.

As can be seen from Table I, minoxidil sulfate, a compound of the present invention, was significantly better than minoxidil (the corresponding N-oxide) in inducing hair growth at 20 mg/kg, and was equipotent to or better than minoxidil at 40 mg/kg. The data further indicates that this hair growth effect is exhibited when minoxidil is topically applied.

## Example 4

Topical minoxidil sulfate solution was applied to a group of 6 stumptail Macque monkeys daily, 7 days/week. The topical minoxidil sulfate solution was prepared by dissolving 750 mg of minoxidil sulfate in 50 ml of a 94:6 volume:volume solution of acetone:N-methylpyrrolidone. A second group of 6 animals served as controls and received vehicle only. the volume per application was 250 microliters applied to the frontal scalp with an art brush. Hair growth rate within a 2.54 cm (1 inch) square area of treated scalp was assessed by quantitatively clipping the hair at successive intervals of 42 days, 21 days and 21 days and weighing the residue. The results are set forth in Table II.

A statistically significant increase in hair growth is seen by the third collection. These data clearly show that minoxidil sulfate stimulates hair growth following topical application to the scalp of balding monkeys.

TABLE I

| Example | Treatment | Score of Photos 2+[1] | Score of Photos 1+[2] |
|---|---|---|---|
| 1 | Minoxidil, 10 mg/kg | 1/5 | 2/5 |
| | Minoxidil, 20 mg/kg | 4/5 | 4/5 |
| | Control | 0/5 | 1/5 |
| 2 | Minoxidil Sulfate, 20 mg/kg | 3/5 | 4/5 |
| | Minoxidil Sulfate, 40 mg/kg | 1/5 | 3/5 |
| | Minoxidil, 20 mg/kg | 0/5 | 1/5 |
| | Minoxidil, 40 mg/kg | 1/5 | 3/5 |
| 3 | Minoxidil Sulfate, 5 mg/kg | 1/5 | 2/5 |
| | Minoxidil Sulfate, 10 mg/kg | 1/5 | 1/5 |
| | Minoxidil Sulfate, 20 mg/kg | 1/5 | 2/5 |
| | Minoxidil Sulfate, 40 mg/kg | 0/5 | 3/5 |
| | Minoxidil, 20 mg/kg | 0/5 | 5/5 |
| | Minoxidil, 40 mg/kg | 0/5 | 1/5 |
| | Minoxidil, 60 mg/kg | 0/5 | 1/5 |
| | Minoxidil, 80 mg/kg | 0/5 | 1/5 |
| | Topical Minoxidil Sulfate, 20 mg/kg | 0/5 | 2/5 |
| | Topical Minoxidil Sulfate, 40 mg/kg | 1/5 | 2/5 |
| | Control | 0/5 | 0/5 |

[1]Number of animals with at least 2 of 5 photos showing positive hair growth.

[2]Number of animals with at least 1 of 5 photos showing positive hair growth.

TABLE II

| Collection | Treatment | N | Mean wt. (uG/day) | T | Prob |
|---|---|---|---|---|---|
| 1 | Minoxidil Sulfate | 6 | 76 ±20 | 0.2 | 0.84 |
| 1 | Vehicle | 6 | 68 ±35 | | |
| 2 | Minoxidil Sulfate | 6 | 313 ±67 | 1.3 | 0.21 |
| 2 | Vehicle | 6 | 195 ±59 | | |
| 3 | Minoxidil Sulfate | 6 | 343 ±52 | 2.9 | 0.02 |
| 3 | Vehicle | 5 | 167 ±32 | | |

**Claims**

1. Use of a compound of formula I

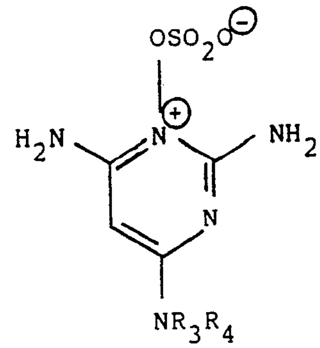

I

wherein either $R_3$ and $R_4$ are independently selected from hydrogen, lower alkyl, lower alkenyl, lower aralkyl and lower cycloalkyl, or $NR_3R_4$ is a heterocyclic moiety selected from aziridinyl, azetidinyl, pyrrolidinyl, piperidino, hexahydroazepinyl, heptamethylenimino, octamethylenimino, morpholino and 4-(lower alkyl)piperazinyl, the heterocyclic moiety optionally having, on its carbon atoms, up to 3 substituents selected from lower alkyl, hydroxy and alkoxy; or a pharmacologically-acceptable acid addition salt thereof;

for the manufacture of a medicament for use in arresting or reversing male pattern baldness in a human, or for use in increasing the rate of terminal hair growth in a mammal.

2. Use according to claim 1, wherein the compound is minoxidil sulfate, i.e. 2,6-diamino-4-(1-piperidinyl)-1-(sulfoxy)-pyrimidinium hydroxide, inner salt.

3. Use according to claim 1 or claim 2, wherein the mammal is a human and the medicament comprises a compound of formula I and a carrier adapted to topical application.

**Patentansprüche**

1. Verwendung einer Verbindung der Formel I

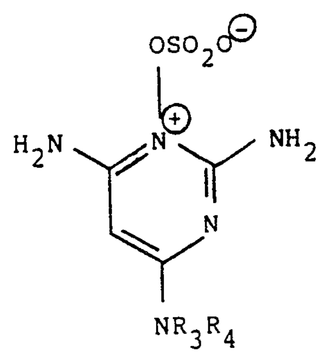

I

worin entweder $R_3$ und $R_4$ unabhängig voneinander aus Wasserstoff, niedrigem Alkyl, niedrigem Alkenyl, niedrigem Aralkyl und niedrigem Cycloalkyl gewählt sind oder $NR_3R_4$ ein heterocyclischer Rest, gewählt aus Aziridinyl, Azetidinyl, Pyrrolidinyl, Piperidino, Hexahydroazepinyl, Heptamethylenimino, Octamethylenimino, Morpholino und 4-(niedrig Alkyl)-piperazinyl ist, wobei der heterocyclische Rest gegebenenfalls an seinen Kohlenstoffatomen bis zu 3 Substituenten, gewählt aus niedrig Alkyl, Hydroxy und Alkoxy trägt, oder einem pharmakologisch verträglichen Säureadditionssalz davon, zur Herstellung eines Pharmazeutikums zur Verwendung bei dem Aufhalten oder Zurückbilden der männlichen Glatzenbildung bei Menschen oder zur Verwendung bei der Erhöhung der Haarwachstumsrate beim Säuger.

2. Verwendung nach Anspruch 1, worin die Verbindung Minoxidilsulfat, d.i. 2,6-Diamino-4-(1-piperidinyl)-1-(sulfoxy)-pyrimidiniumhydroxid, inneres Salz ist.

3. Verwendung nach Anspruch 1 oder 2, worin der Säuger ein Mensch ist und das Pharmazeutikum eine Verbindung der Formel I und einen Träger, welcher für die topische Anwendung adaptiert ist, enthält.

**Revendications**

1. Utilisation d'un composé de formule I

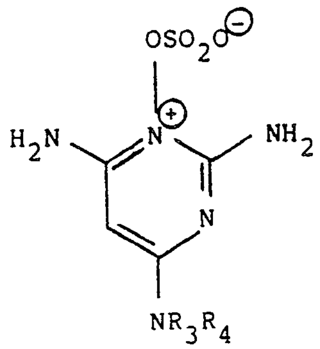

I

dans laquelle $R_3$ et $R_4$ sont choisis, indépendamment, entre l'hydrogène, des groupes alkyle inférieur, alcényle inférieur, aralkyle inférieur et cycloalkyle inférieur, ou bien $NR_3R_4$ représente un groupement hétérocyclique choisi entre des groupes aziridinyle, azétidinyle, pyrrolidinyle, pipéridino, hexahydro-azépinyle, heptaméthylène-imino, octaméthylène-imino, morpholino et 4-(alkyle inférieur)-pipérazinyle, le groupement hétérocyclique portant facultativement, sur ses atomes de carbone, jusqu'à 3 substituants choisis entre des groupes alkyle inférieur, hydroxy et alkoxy; ou d'un de ses sels d'addition d'acides pharmacologiquement acceptables;

pour la production d'un médicament destiné à être utilisé pour arrêter ou inverser l'alopécie hippocratique chez l'homme, ou bien destiné à être utilisé pour accroître la vitesse de croissance des cheveux terminaux chez un mammifère.

2. Utilisation suivant la revendication 1, dans laquelle le composé est le sulfate de minoxidil, c'est-à-dire l'hydroxyde de 2,6-diamino-4-(1-pipéridinyl)-1-(sulfoxy)-pyrimidinium, sel interne.

3. Utilisation suivant la revendication 1 ou la revendication 2, dans laquelle le mammifère est un être humain et le médicament comprend un composé de formule I et un véhicule permettant une application topique.